# EUROPEAN PATENT APPLICATION

(11) **EP 3 824 891 A1**
(43) Date of publication of application: **26.05.2021**
(21) Application number: 19837200.5
(22) Date of filing: 18.07.2019
(51) Int. Cl.: A61K 31/7088, A61K 9/127, A61K 9/133, A61K 47/18, A61K 48/00, A61P 35/00, A61P 37/04

(54) **LIPID PARTICLE CONTAINING A-TYPE CPG OLIGODEOXYNUCLEOTIDE**

(30) Priority: 19.07.2018 JP 2018135787
(71) Applicant: The Research Foundation for Microbial Diseases of Osaka University, Suita-shi, Osaka 565-0871 (JP); Osaka University, Suita-shi, Osaka 565-0871 (JP); Teikyo University, Tokyo 173-8605 (JP)
(72) Inventor: AOSHI, Taiki, Suita-shi, Osaka 565-0871 (JP); KOYAMA, Shohei, Suita-shi, Osaka 565-0871 (JP); SUZUKI, Ryo, Tokyo 173-8605 (JP)
(74) Representative: Hoffmann Eitle
(86) International application number: PCT/JP2019/028269
(87) International publication number: WO 2020/017590

(57) **Abstract**

An object is to provide a technology that can stably exhibit the effects of A-type CpG oligodeoxynucleotides. The object can be achieved by a lipid particle comprising an A-type CpG oligodeoxynucleotide.

SEQUENCE LISTING

<110>  THE RESEARCH FOUNDATION FOR MICROBIAL DISEASES 0F OSAKA UNIVERSITY
       OSAKA UNIVERSITY
       TEIKYO UNIVERSITY

<120>  A lipid particle comprising A type CpG oligodeoxynucleotide

<130>  P19-118WO

<150>  JP 2018-135787
<151>  2018-07-19

<160>  2

<170>  PatentIn version 3.5

<210>  1
<211>  20
<212>  DNA
<213>  Artificial Sequence

<220>
<223>  D35

<400>  1
   ggtgcatcga tgcagggggg                                                    20


<210>  2
<211>  12
<212>  DNA
<213>  Artificial Sequence

<220>
<223>  D35 core

<400>  2
tgcatcgatg ca                                                           12

SEQUENCE LISTING

<110>  THE RESEARCH FOUNDATION FOR MICROBIAL DISEASES 0F OSAKA UNIVERSITY
       OSAKA UNIVERSITY
       TEIKYO UNIVERSITY

<120>  A lipid particle comprising A type CpG oligodeoxynucleotide

<130>  P19-118WO

<150>  JP 2018-135787
<151>  2018-07-19

<160>  2

<170>  PatentIn version 3.5

<210>  1
<211>  20
<212>  DNA
<213>  Artificial Sequence

<220>
<223>  D35

<400>  1
   ggtgcatcga tgcagggggg                                                    20


<210>  2
<211>  12
<212>  DNA
<213>  Artificial Sequence

<220>
<223>  D35 core

<400>  2
tgcatcgatg ca                                                           12

## Description

### Technical Field

The present invention relates to a lipid particle, a method for producing the lipid particle, and a medicament, a reagent, an interferon production promoter, an anticancer agent, and the like, all of which comprise the lipid particle.

### Background Art

CpG oligodeoxynucleotides induce innate immune responses in a Toll-like receptor (TLR) 9-dependent manner, and are expected as vaccine adjuvants that enhance the immune effect of a vaccine when administered together with a vaccine antigen, or as immunomodulators due to innate immune responses evoked by themselves. A/D type, B/K type, C type, P type, etc., are known as CpG oligodeoxynucleotides.

The B/K type is soluble in physiological saline and is known to mainly induce interleukin (IL)-6 production. This type is already being clinically applied as a promising nucleic acid medicine. The C type and P type can induce IFN-α in addition to IL-6; however, the degree of induction by the C type is weak. All of these three types have a high proportion of phosphorothioate skeletons, and their toxicity is a concern.

On the other hand, the A/D type is insoluble in physiological saline, but is known to strongly induce interferon (IFN)-α production. This type has different biological activities from those of the B/K type, C type, and P type. Further, the A/D type has a low proportion of phosphorothioate skeletons and is considered to have a low toxicity problem.

Strong IFN-α induction by the A/D type is closely related to higher-order structures of this type. A/D-type poly-G tails form a guanine-quadruplex DNA structure in a salt solution, resulting in the formation of nanoparticles/aggregates. It has been repeatedly reported that the formation of aggregates is essential for high IFN-α production by the A/D type, which is a major barrier to the clinical application of A/D-type ODN. This is because the voluntary occurrence of A/D-type multimerization leads to uncontrolled aggregation and sedimentation, resulting in large differences between products and difficulty in administration. Thus, it is difficult to stably exhibit the effect of the A/D type.

In order to solve this problem, PTL 1 discloses a technique of adding poly-A to the end of the A/D type. However, the addition of poly-A increases the number of bases in the nucleic acid, which causes problems such as difficulty in synthesis and soaring synthesis costs. Moreover, poly-A has a phosphorothioate skeleton; however, from the viewpoint of toxicity, poly-A with a lower proportion of phosphorothioate skeletons is desired.

### Citation List

### Patent Literature

PTL 1: WO2016/103703

### Summary of Invention

### Technical Problem

An object of the present invention is to provide a technology that can stably exhibit the effects of A-type CpG oligodeoxynucleotides.

### Solution to Problem

As a result of extensive research, the present inventors found that a lipid particle comprising an A-type CpG oligodeoxynucleotide can stably exhibit the effects of the A type, such as IFN-α production-inducing action. Surprisingly, the present inventors found that the effects of A-type CpG oligodeoxynucleotides were enhanced by forming them into lipid particles. As a result of further research based on these findings, the present invention has been completed.

Specifically, the present invention includes the following embodiments.
Item 1.
   A lipid particle comprising an A-type CpG oligodeoxynucleotide.
Item 2.
   The lipid particle according to Item 1, wherein the lipid particle comprises an outer layer and an ion complex placed inside the outer layer.
Item 3.
   The lipid particle according to Item 2, wherein the ion complex contains an A-type CpG oligodeoxynucleotide.
Item 4.
   The lipid particle according to Item 2 or 3, wherein the outer layer is a lipid monolayer membrane in which amphipathic lipids are arranged with hydrophilic parts facing outward.
Item 5.
   The lipid particle according to any one of Items 1 to 4, wherein a water-soluble polymer-modified lipid is contained in an amount of 0 to 10 mass% based on 100 mass% of lipids constituting the lipid particle.
Item 6.
   The lipid particle according to any one of Items 1 to 5, wherein the ratio (N/P) of the number of nitrogen atoms (N) in the lipids constituting the lipid particle to the number of phosphorus atoms (P) in a nucleic acid containing the A-type CpG oligodeoxynucleotide is 2.5 or more.
Item 7.
   The lipid particle according to any one of Items 1 to 6, comprising a cationic lipid.
Item 8.
   The lipid particle according to Item 7, wherein the cationic lipid is at least one member selected from the group consisting of 1,2-dioleoyloxy-3-trimethylammonium-propane (DOTAP) and 1,2-dioleyloxy-3-trimethylammonium-propane (DOTMA).
Item 9.
   A method for producing a lipid particle, comprising the step of mixing a lipid-containing alcohol solution and an A-type CpG oligodeoxynucleotide-containing aqueous solution.
Item 10.
   The production method according to Item 9, wherein the step is performed in a reaction system using a microchannel.
Item 11.
   A medicament comprising the lipid particle according to any one of Items 1 to 8.
Item 12.
   A reagent comprising the lipid particle according to any one of Items 1 to 8.
Item 13.
   An immunostimulant comprising the lipid particle according to any one of Items 1 to 8.
Item 13A.
   An immunostimulation method comprising administering the lipid particle according to any one of Items 1 to 8 to a subject in need of immunostimulation.
Item 13B.
   The lipid particle according to any one of Items 1 to 8 for use as an immunostimulant.
Item 13C.
   Use of the lipid particle according to any one of Items 1 to 8 for producing an immunostimulant.
Item 13D.
   Use of the lipid particle according to any one of Items 1 to 8 as an immunostimulant.
Item 14.
   An anticancer agent comprising the lipid particle according to any one of Items 1 to 8.
Item 14A.
   A method for treating cancer, comprising administering the lipid particle according to any one of Items 1 to 8 to a subject in need of cancer treatment.
Item 14B.
   The lipid particle according to any one of Items 1 to 8 for use as an anticancer agent.
Item 14C.
   Use of the lipid particle according to any one of Items 1 to 8 for producing an anticancer agent.
Item 14D. Use of the lipid particle according to any one of Items 1 to 8 as an anticancer agent.

### Advantageous Effects of Invention

The present invention provides a technology that can exhibit the effects of A-type CpG oligodeoxynucleotides stably and more strongly, specifically lipid particles comprising A-type CpG oligodeoxynucleotides. The lipid particles can be used as medicaments, reagents, immunostimulants, anticancer agents, and the like.

### Brief Description of Drawings

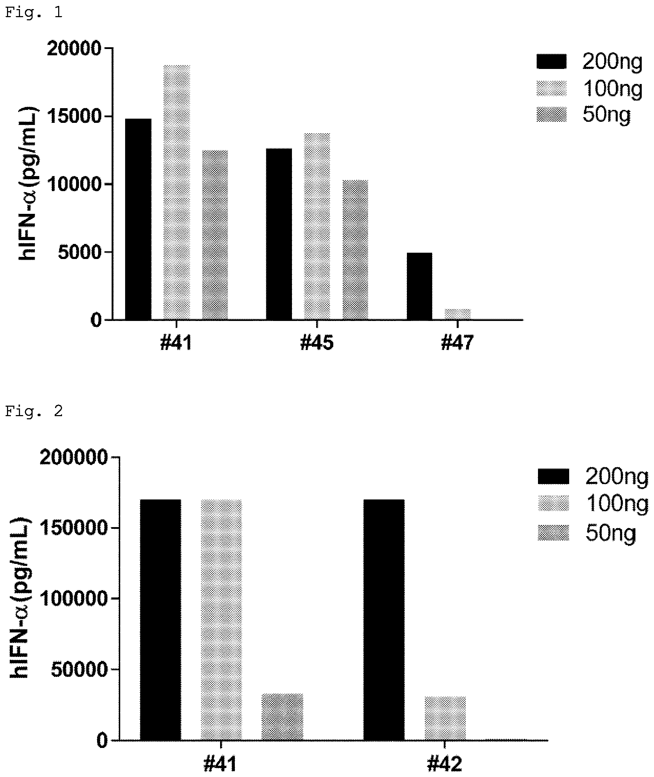

Fig. 1 shows the results of examining the induction of IFN-α production in human PBMC by lipid nanoparticles containing an A-type CpG oligodeoxynucleotide (D35: SEQ No. 1) containing a cationic lipid (#41: DOTAP, #45: DOTMA, or #47: DODAP) (Test Example 1). The vertical axis shows the IFN-α concentration of the culture supernatant. The legend shows the amount of lipid nanoparticles in terms of D35 added to human PBMC culture medium.
Fig. 2 shows the results of examining the induction of IFN-α production in human PBMC by D35-containing lipid nanoparticles with different N/P ratios (#41: 3 or #42: 1) (Test Example 2). The vertical axis shows the IFN-α concentration of the culture supernatant. The legend shows the amount of lipid nanoparticles in terms of D35 added to human PBMC culture medium.

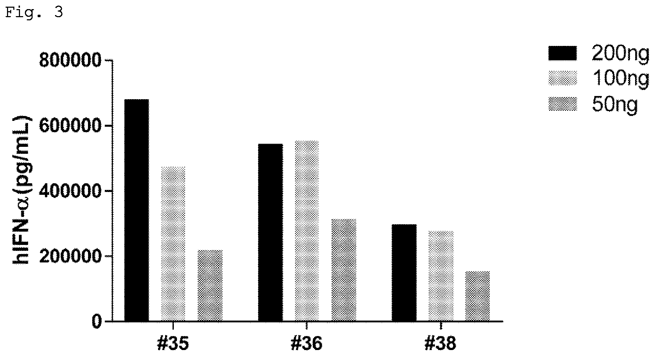

Fig. 3 shows the results of examining the induction of IFN-α production in human PBMC by D35-containing lipid nanoparticles obtained by varying a dialysis solution with (or without) the addition of a pH-sensitive lipid (DOPE). The vertical axis shows the IFN-α concentration of the culture supernatant. The legend shows the amount of lipid nanoparticles in terms of D35 added to human PBMC culture medium. The combination of the presence of DOPE and the dialysis solution is as follows: #35: DOPE-free/phosphate buffered saline (PBS) dialysis, #36: DOPE-free/5% glucose dialysis, and #38: DOPE-containing/5% glucose dialysis.

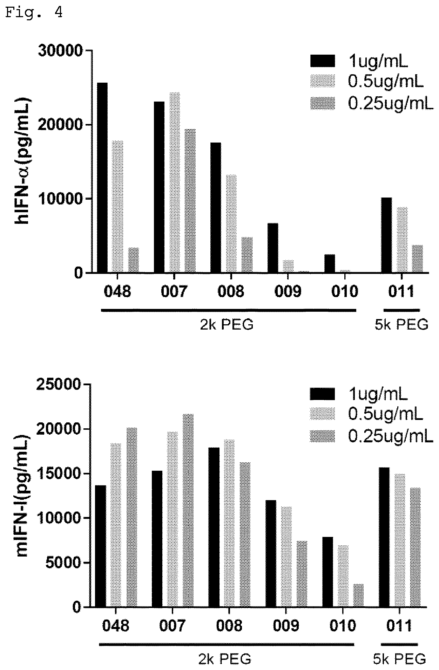

Fig. 4 shows the results of examining the induction of IFN-α production in human PBMC or mouse spleen cells by D35-containing lipid nanoparticles obtained by varying the content and PEG chain length of DSPE-PEG-OMe (Test Example 4). The upper graph shows the results of human PBMC (the vertical axis shows the IFN-α concentration of the culture supernatant). The lower graph shows the results of mouse spleen cells (the vertical axis shows the IFN-I concentration of the culture supernatant). The legend shows the medium final concentration of lipid nanoparticles in terms of D35 added to human PBMC culture medium. The combination of the content and PEG chain length of DSPE-PEG-OMe is as follows: 2k PEG: 0.0 mass% (048), 2k PEG: 0.5 mass% (007), 2k PEG: 1.0 mass% (008), 2k PEG: 3.0 mass% (009), 2k PEG: 6.0 mass% (010), and 5k PEG 0.5 mass% (011).

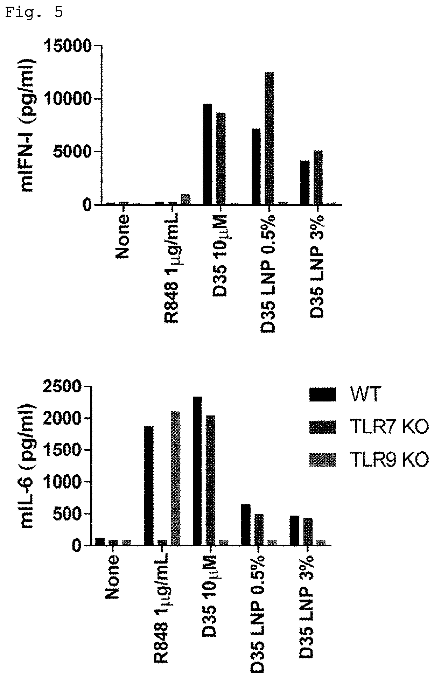

Fig. 5 shows the results of examining the TLR9 dependency of D35-containing lipid nanoparticles (Test Example 5). The upper graph shows the results of examining the IFN-I concentration, and the lower graph shows the results of examining the IL-6 concentration. "D35LNP" indicates the case of adding D35-containing lipid nanoparticles (medium final concentration: 1 ug/mL), and "0.5%" and "3"%" indicate the DSPE-PEG-OMe contents per 100 mass% of lipids in the lipid particles. The values ("1 ug/mL" and "10uM") for R848 and D35 indicate the medium final concentrations.

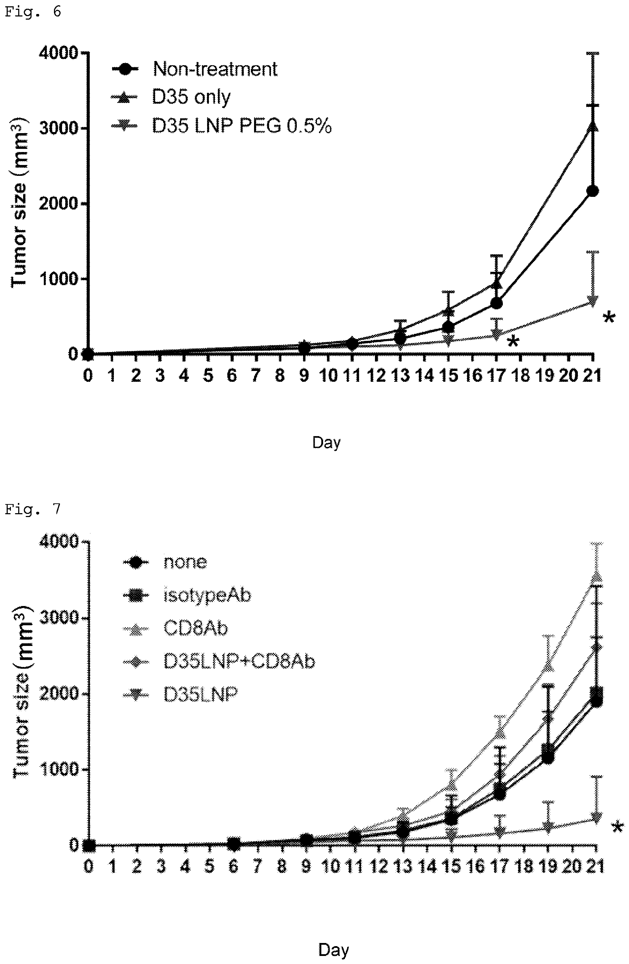

Fig. 6 shows the results of examining the anticancer effect of D35-containing lipid nanoparticles (Test Example 6). The vertical axis shows the tumor size, and the horizontal axis shows the number of days elapsed after subcutaneous transplantation of tumor cells. The legend indicates the presence and type of pharmaceutical agent administered. Each plot shows the average value (n = 5), and the bars on each plot show the standard deviation. * indicates that the P value (Dunn's multiple comparisons test) for no pharmaceutical agent ("Non-treatment") is less than 0.05.
Fig. 7 shows the results of examining the anticancer effect of D35-containing lipid nanoparticles (Test Example 7). The vertical axis shows the tumor size, and the horizontal axis shows the number of days elapsed after subcutaneous transplantation of tumor cells. The legend indicates the presence and types of pharmaceutical agent and antibody administered. Each plot shows the average value (n = 5), and the bars on each plot show the standard deviation. * indicates that the P value (Dunn's multiple comparisons test) for no pharmaceutical agent ("none") is less than 0.05.

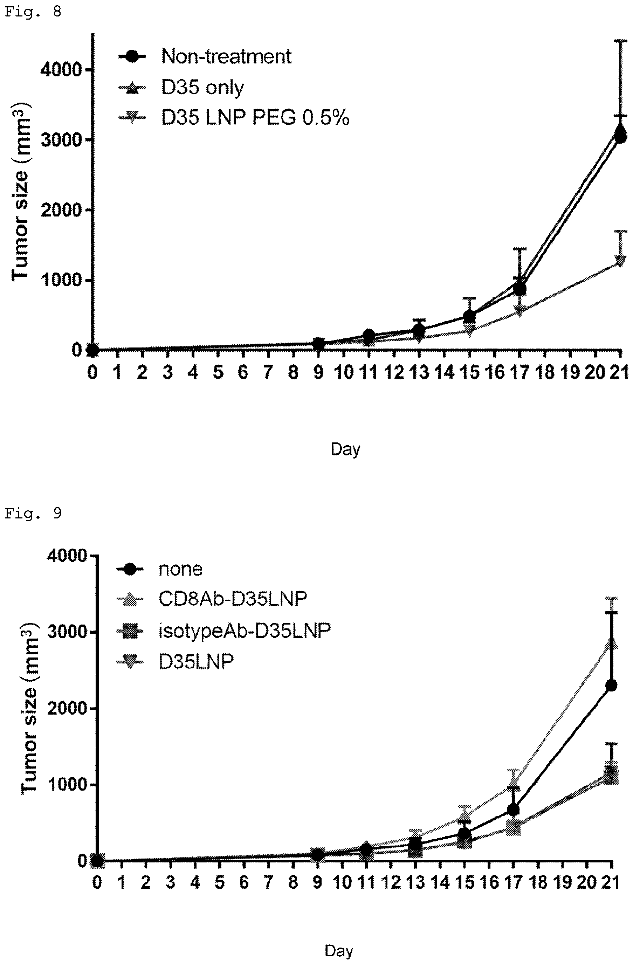

Fig. 8 shows the results of examining the anticancer effect of D35-containing lipid nanoparticles (Test Example 9). The vertical axis shows the tumor size, and the horizontal axis shows the number of days elapsed after subcutaneous transplantation of tumor cells. The legend indicates the presence and type of pharmaceutical agent administered. Each plot shows the average value (n = 5), and the bars on each plot show the standard deviation.
Fig. 9 shows the results of examining the anticancer effect of D35-containing lipid nanoparticles (Test Example 11). The vertical axis shows the tumor size, and the horizontal axis shows the number of days elapsed after subcutaneous transplantation of tumor cells. The legend indicates the presence and types of pharmaceutical agent and antibody administered. Each plot shows the average value (n = 5), and the bars on each plot show the standard deviation.

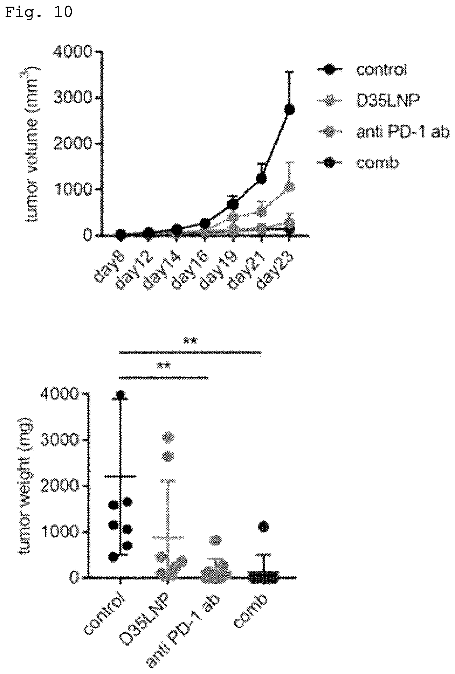

Fig. 10 shows the results of examining the anticancer effect of D35-containing lipid nanoparticles (Test Example 12). In the upper graph, the vertical axis shows the tumor size, the horizontal axis shows the number of days elapsed after subcutaneous transplantation of tumor cells, and the legend indicates the presence and type of pharmaceutical agent administered. In the lower graph, the vertical axis shows the tumor weight, and the horizontal axis shows the presence and type of pharmaceutical agent. "comb" shows the combination of D35-containing lipid nanoparticles and anti-PD-1 antibody. In the upper graph, each plot shows the average value (n = 8), and the bars on each plot show the standard deviation. In the lower graph, each plot shows the data for each sample, the horizontal line in the center of the bar shows the median value, the horizontal lines at both ends show the standard deviation, and ** indicates that the P-value (one-way ANOVA) is less than 0.01.

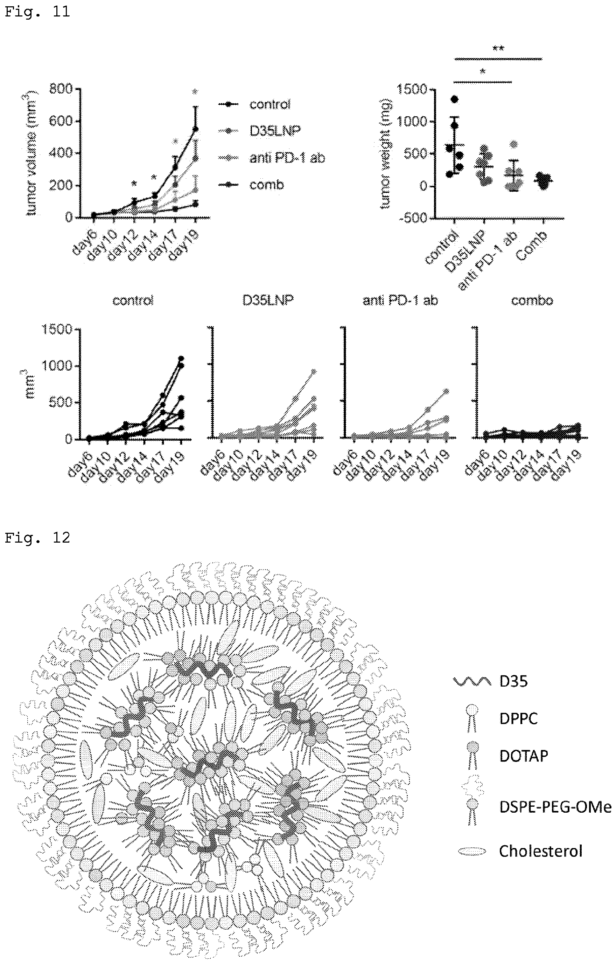

Fig. 11 shows the results of examining the anticancer effect of D35-containing lipid nanoparticles (Test Example 12). In the upper-left graph, the vertical axis shows the tumor size, the horizontal axis shows the number of days elapsed after subcutaneous transplantation of tumor cells, and the legend indicates the presence and type of pharmaceutical agent administered. In the upper-right graph, the vertical axis shows the tumor weight, and the horizontal axis shows the presence and type of pharmaceutical agent. In the lower graphs, the vertical axis shows the tumor size, and the horizontal axis shows the number of days elapsed after subcutaneous transplantation of tumor cells. The presence and type of pharmaceutical agent administered are shown above each graph. "combs" shows the combination of D35-containing lipid nanoparticles and anti-PD-1 antibody. In the upper-left graph, each plot shows the average value (n = 7), and the bars on each plot show the standard deviation. In the upper-right graph, each plot shows the data for each sample, the horizontal line in the center of the bar shows the median value, the horizontal lines at both ends show the standard deviation, * indicates that the P-value (one-way ANOVA) is less than 0.05, and ** indicates that the P-value is less than 0.01.
Fig. 12 is a schematic diagram showing a preferred embodiment of the liquid particle of the present invention. In the figures showing phospholipids, cationic lipids, and PEG-lipids, the circle parts indicate hydrophilic parts, and double bars indicate hydrophobic parts. In the figure showing PEG-lipids, the wavy bars extending from the circle parts indicate PEG chains.

### Description of Embodiments

The terms "containing" and "comprising" as used herein include the concepts of "containing," "comprising," "substantially consisting of," and "consisting of."

### 1. Lipid Particle

In one embodiment, the present invention relates to a lipid particle comprising an A-type CpG oligodeoxynucleotide (also referred to herein as "the lipid particle of the present invention"). This is described below.

The A-type CpG oligodeoxynucleotide (ODN) is ODN having the function of inducing the production of type I IFN (particularly IFN-α) from plasmacytoid dendritic cells, and is not particularly limited as long as it has this function.

Typical A-type CpG ODN contains a core part comprising a palindromic sequence centered on the central base sequence (CG (unmethylated)), and a G-oligomer part arranged on one side of the core part.

The base length of the core part is not particularly limited, but is, for example, 5 to 30, preferably 7 to 20, more preferably 9 to 15, and even more preferably 10 to 14.

The base sequence of the core part is not particularly limited, as long as it is a palindromic sequence centered on the central base sequence (CG). Examples include the base sequence represented by SEQ ID NO: 2.

All bonds between nucleosides in the core part are phosphodiester bonds.

The base length of the G oligomer is not particularly limited, and is 2 to 10, for example. The base length is preferably 3 to 8, more preferably 5 to 7, and even more preferably 6.

The G oligomer is preferably placed on the 3'-side of the core part.

In the core part, another base may not be present or may be present on the opposite side of the side on which the G oligomer is placed. The other base is preferably, for example, a base/base sequence consisting of G. The base length of the base/base sequence is not particularly limited, and is, for example, 1 to 10, preferably 1 to 4, and more preferably 2.

In the parts other than the core part (the G-oligomer part and the other base part), it is preferable that several (e.g., 2 to 5, preferably 2 to 3, more preferably 2) nucleosides from the end (3'-end or 5'-end) are phosphorothioate-bonded to adjacent nucleosides. Bond portions other than phosphorothioate bonds are generally phosphodiester bonds.

A-type CpG oligodeoxynucleotides may be used singly or in combination of two or more.

The structure of the lipid particle of the present invention is not particularly limited, as long as it is a complex of an A-type CpG oligodeoxynucleotide and lipids. Examples of the lipid particle of the present invention include particles in which lipids including amphipathic lipids form an outer layer, and the amphipathic lipids are arranged with their hydrophilic parts facing outward. Examples of such particles include particles having an outer layer comprising a lipid monolayer membrane (lipid single membrane) and particles having an outer layer comprising a lipid bilayer membrane (lipid double membrane), preferably particles having an outer layer comprising a lipid monolayer membrane, and more preferably particles having an outer layer comprising a lipid monolayer membrane in which amphipathic lipids are arranged with their hydrophilic parts facing outward. The inner layer of the particle may comprise a uniform aqueous phase or oil phase, but preferably contains one or more ion complexes. The ion complex preferably contains an A-type CpG oligodeoxynucleotide, and more preferably contains an A-type CpG oligodeoxynucleotide in its inner layer. A preferred embodiment of the ionic complex includes a complex of an A-type CpG oligodeoxynucleotide and a cationic lipid, and more preferably a complex with hydrophobically bound cholesterol. In a preferred embodiment of the lipid particle of the present invention, the lipid particle of the present invention comprises an outer layer and one or more ion complexes placed inside the outer layer. Fig. 12 shows a preferred embodiment of the lipid particle of the present invention.

The particle size of the lipid particle of the present invention is not particularly limited. The particle size is preferably nano-scale, and is specifically, for example, 10 to 700 nm, preferably 10 to 500 nm, more preferably 10 to 250 nm, even more preferably 20 to 200 nm, still even more preferably 20 to 150 nm, and particularly preferably 20 to 120 nm.

The lipid particle of the present invention contains amphipathic lipids, such as phospholipids and glycolipids, as particle constituent lipids. The particle constituent lipids may include other lipids. Specific examples of other lipids include sterols, saturated or unsaturated fatty acids, and the like.

Specific examples of phospholipids include phosphatidylcholines such as dilauroylphosphatidylcholine, dimyristoylphosphatidylcholine, dipalmitoylphosphatidylcholine (DPPC), distearoylphosphatidylcholine, dioleoylphosphatidylcholine, dilinoleoylphosphatidylcholine, myristoylpalmitoylphosphatidylcholine, myristoylstearoylphosphatidylcholine, and palmitoylstearoylphosphatidylcholine; phosphatidylglycerols such as dilauroylphosphatidylglycerol, dimyristoylphosphatidylglycerol, dipalmitoylphosphatidylglycerol, distearoylphosphatidylglycerol, dioleoylphosphatidylglycerol, dilinoleoylphosphatidylglycerol, myristoylpalmitoylphosphatidylglycerol, myristoylstearoylphosphatidylglycerol, and palmitoylstearoylphosphatidylglycerol; phosphatidylethanolamines such as dilauroylphosphatidylethanolamine, dimyristoylphosphatidylethanolamine, dipalmitoylphosphatidylethanolamine, distearoylphosphatidylethanolamine (DSPE), dioleoylphosphatidylethanolamine (DOPE), dilinoleoylphosphatidylethanolamine, myristoylpalmitoylphosphatidylethanolamine, myristoylstearoylphosphatidylethanolamine, and palmitoylstearoylphosphatidylethanolamine; cationic lipids such as 1,2-dioleoyloxy-3-trimethylammonium-propane (DOTAP), 1,2-dioleyloxy-3-trimethylammonium-propane (DOTMA), and 1,2-dioleoyloxy-3-dimethylammonium-propane (DODAP); phosphatidylserine; lysophosphatidylcholine; phosphatidic acid; phosphatidylinositol; sphingomyelin; cardiolipin; egg yolk lecithin; soybean lecithin; and hydrogenated additives thereof.

Specific examples of glycolipids include glyceroglycolipids such as diglycosyl diglyceride, digalactosyl diglyceride, galactosyl diglyceride, and glycosyl diglyceride; glycosphingolipids such as galactosyl cerebroside and ganglioside; stearyl glucoside, esterified stearyl glycoside, and the like.

Specific examples of sterols include cholesterol, cholesteryl hemisuccinate, lanosterol, dihydrolanosterol, desmosterol, dihydrocholesterol, phytosterol, phytosterol, stigmasterol, zymosterol, ergosterol, sitosterol, campesterol, brassicasterol, and the like. In particular, due to the action of stabilizing the liposome membrane and regulating the fluidity of the liposome membrane, such a sterol is desirably contained as a constituent lipid of the liposome membrane.

Specific examples of saturated or unsaturated fatty acids include saturated or unsaturated fatty acids having 10 to 22 carbon atoms, such as decanoic acid, myristic acid, palmitic acid, stearic acid, arachidonic acid, oleic acid, and docosanoic acid.

The lipids constituting the lipid particle of the present invention may be used singly or in combination of two or more.

The lipid particle of the present invention preferably contains an amphipathic lipid and a sterol as particle constituent lipids.

Examples of amphipathic lipids preferably include phospholipids, and more preferably cationic lipids, phosphatidylcholine, phosphatidylethanolamine, and the like. In a preferred embodiment, the lipid particle of the present invention contains a cationic lipid, more preferably a cationic lipid and other phospholipids (preferably phosphatidylcholine, phosphatidylethanolamine, etc.). Preferred examples of cationic lipids include 1,2-dioleoyloxy-3-trimethylammonium-propane (DOTAP), 1,2-dioleyloxy-3-trimethylammonium-propane (DOTMA), and the like. Examples of other phospholipids include phospholipids in which hydrocarbon groups in the hydrophobic portions preferably have, for example, 10 to 25 carbon atoms, preferably 12 to 21 carbon atoms, and more preferably 14 to 18 carbon atoms (e.g., dipalmitoylphosphatidylcholine (DPPC) and dioleoylphosphatidylethanolamine (DOPE)).

The content of the amphipathic lipid is not particularly limited, but is, for example, 20 to 95 mass%, preferably 40 to 90 mass%, more preferably 50 to 85 mass%, even more preferably 60 to 80 mass%, still even more preferably 65 to 75 mass%, and particularly preferably 67 to 73 mass%, per 100 mass% of the lipids constituting the lipid particle of present invention.

When a cationic lipid is contained as the amphipathic lipid, the content of the cationic lipid is not particularly limited, but is, for example, 10 to 75 mass%, preferably 20 to 70 mass%, more preferably 30 to 65 mass%, even more preferably 40 to 60 mass%, still even more preferably 45 to 55 mass%, and particularly preferably 47 to 53 mass%.

When a phospholipid other than cationic lipids is contained as the amphipathic lipid, the content of the other phospholipid is not particularly limited, but is, for example, 5 to 50 mass%, preferably 10 to 30 mass%, more preferably 15 to 25 mass%, and even more preferably 17 to 23 mass%.

Preferred examples of sterols include cholesterol.

The content of sterol is not particularly limited, but is, for example, 15 to 60 mass%, preferably 20 to 40 mass%, more preferably 25 to 35 mass%, and even more preferably 27 to 33 mass%, per 100 mass% of the lipids constituting the lipid particle of present invention.

It is desirable that the lipid particle of the present invention contains a water-soluble polymer-modified lipid as a particle constituent lipid, in addition to the above-mentioned preferable particle constituent lipids (amphipathic lipids and sterols). The water-soluble polymer is not particularly limited, and examples include polyethylene glycol (PEG) chains. The molecular weight of the water-soluble polymer is not particularly limited, but is, for example, 200 to 10000, preferably 500 to 7000, more preferably 500 to 4000, even more preferably 1000 to 3000, and still even more preferably 1500 to 2500. Examples of the lipid modified with a water-soluble polymer preferably includes amphipathic lipids, more preferably phospholipids, and even more preferably phosphatidylethanolamine.

The content of the water-soluble polymer-modified lipid is not particularly limited, but is, for example, 0 to 10 mass%, preferably 0 to 5 mass%, more preferably 0 to 2 mass%, even more preferably 0 to 1 mass%, still even more preferably 0.2 to 1 mass%, and particularly preferably 0.3 to 0.7 mass%, per 100 mass% of the lipids constituting the lipid particle of present invention.

The ratio (N/P) of the number of nitrogen atoms (N) in the lipids constituting the lipid particle to the number of phosphorus atoms (P) in the nucleic acid containing the A-type CpG oligodeoxynucleotide is preferably 2.5 or more, more preferably 2.5 to 5, even more preferably 2.5 to 4, and still even more preferably 2.5 to 3.5.

In addition to the above components, the lipid particle of the present invention may contain one or more other components. Examples of other components include membrane stabilizers, charged substances, antioxidants, membrane proteins, polyethylene glycol (PEG), antibodies, peptides, sugar chains, and the like.

An antioxidant can be contained to prevent the oxidation of the membrane, and is used as a membrane constituent as necessary. Examples of the antioxidant used as a membrane constituent include butylated hydroxytoluene, propyl gallate, tocopherol, tocopherol acetate, concentrated mixed tocopherol, vitamin E, ascorbic acid, L-ascorbic acid stearic acid ester, ascorbic acid palmitate, sodium hydrogen sulfite, sodium sulfite, sodium edetate, erythorbic acid, citric acid, and the like.

A membrane protein can be contained for the purpose of adding functions to the membrane or stabilizing the structure of the membrane, and is used as a membrane constituent as necessary. Examples of membrane proteins include superficial membrane proteins, integral membrane proteins, albumin, recombinant albumin, and the like.

The content of one or more other components is, for example, 10% or less, preferably 5% or less, more preferably 2% or less, and even more preferably 1% or less, based on 100 mass% of the lipid particle of the present invention.

The lipid particle of the present invention can be produced by or according to a known method for lipid particles. The lipid particle of the present invention can be preferably produced by a method comprising the step of mixing a lipid-containing alcohol solution and an A-type CpG oligodeoxynucleotide-containing aqueous solution (step 1).

The alcohol used as the solvent for the alcohol solution is not particularly limited, as long as it is an alcohol that can dissolve phospholipids. Ethanol is preferably used as the alcohol.

In addition to a water-soluble drug and water as a solvent, the aqueous solution may contain a cryoprotectant, such as sugar (e.g., sucrose or trehalose) or polyethylene glycol.

The mixing ratio of the aqueous solution to the alcohol solution (acidic aqueous solution/alcohol solution, v/v) is, for example, 20/1 to 1/1, and preferably 4/1 to 2/1.

Mixing is not particularly limited, as long as lipid particles can be formed; however, the mixture is generally vigorously stirred with a vortex or the like. Alternatively, when mixing is performed in a reaction system using a microchannel, the mixture is mixed in the reaction system.

Step 1 is generally carried out at room temperature or under heating. The temperature of step 1 is, for example, 15°C to 60°C, and preferably 15°C to 45°C.

### 2. Use

The lipid particle of the present invention can exhibit the effects of A-type CpG oligodeoxynucleotides stably and more strongly. Therefore, the lipid particle of the present invention can be used for various applications utilizing the effects of A-type CpG oligodeoxynucleotides (e.g., type I INF production-inducing action and INF-α production-inducing action). For example, the lipid particle of the present invention can be used as an active ingredient of medicaments, reagents, and the like (also referred to herein as "the pharmaceutical agent of the present invention"); and more specifically, as an active ingredient of anticancer agents, immunostimulants, and the like. Even more specifically, the lipid particle of the present invention can be used as an active ingredient of type I INF production promoters, INF-α production promoters, Th1-related gene expression promoters, and the like.

The pharmaceutical agent of the present invention is not particularly limited, as long as it contains the lipid particle of the present invention, and may further contain one or more other components as necessary. These other components are not particularly limited, as long as they are pharmaceutically acceptable components. Examples of such other components include components having a pharmacological action, and additives. Examples of additives include bases, carriers, solvents, dispersants, emulsifiers, buffers, stabilizers, excipients, binders, disintegrants, lubricants, thickeners, humectants, colorants, fragrances, chelating agents, and the like.

The lipid particle of the present invention alone can exert the above-mentioned effects. Therefore, the pharmaceutical agent of the present invention can exhibit its desired effects without containing any other component having the above-mentioned effects and/or actions, but may contain one or more other components having a pharmacological action.

The lipid particle of the present invention can be used in combination with other anticancer agents. This combination can provide an enhanced effect. Other anticancer agents are not particularly limited, and various anticancer agents can be used. Examples of anticancer agents include alkylating agents, metabolic antagonists, microtubule inhibitors, antibiotic anticancer agents, topoisomerase inhibitors, platinum drugs, molecular targeted drugs, hormone agents, biological agents, and the like. Preferable examples include metabolic antagonists, antibiotic anticancer agents, platinum drugs, and the like. Examples of alkylating agents include cyclophosphamide, ifosfamide, nitrosourea, dacarbazine, temozolomide, nimustine, busulfan, melphalan, procarbazine, ranimustine, and the like. Examples of metabolic antagonists include enocitabine, carmofur, capecitabine, tegafur, tegafur-uracil, tegafur-gimeracil-oteracil potassium, gemcitabine, cytarabine, cytarabine ocfosfate, nelarabine, fluorouracil, fludarabine, pemetrexed, pentostatin, methotrexate, cladribine, doxifluridine, hydroxycarbamide, mercaptopurine, and the like. Examples of microtubule inhibitors include alkaloid anticancer agents such as vincristine; and taxane anticancer agents such as docetaxel and paclitaxel. Examples of antibiotic anticancer agents include mitomycin C, doxorubicin, epirubicin, daunorubicin, bleomycin, actinomycin D, aclarubicin, idarubicin, pirarubicin, peplomycin, mitoxantrone, amrubicin, zinostatin stimalamer, and the like. Examples of topoisomerase inhibitors include CPT-11, irinotecan, and nogitecan, which have topoisomerase I inhibitory action; and etoposide and sobuzoxane, which have topoisomerase II inhibitory action. Examples of platinum drugs include cisplatin, nedaplatin, oxaliplatin, carboplatin, and the like. Examples of hormone agents include dexamethasone, finasteride, tamoxifen, astrozole, exemestane, ethinylestradiol, chlormadinone, goserelin, bicalutamide, flutamide, prednisolone, leuprorelin, letrozole, estramustine, toremifene, fosfestrol, mitotane, methyltestosterone, medroxyprogesterone, mepitiostane, and the like. Examples of biological drugs include interferon α, interferon β, interferon γ, interleukin 2, ubenimex, dry BCG, and the like. Examples of molecular targeted drugs include nivolumab, pembrolizumab, rituximab, alemtuzumab, trastuzumab, cetuximab, panitumumab, imatinib, dasatinib, nilotinib, gefitinib, erlotinib, temsirolimus, bevacizumab, VEGF trap, sunitinib, sorafenib, tosituzumab, bortezomib, gemutuzumab-ozogamicin, ibritumomab-ozogamicin, ibritumomab tiuxetan, tamibarotene, tretinoin, and the like. In addition to the above-specified molecular targeted drugs, examples include the following molecular targeted drugs: angiogenesis-targeted inhibitors such as human epidermal growth factor receptor 2 inhibitors, epidermal growth factor receptor inhibitors, Bcr-Abl tyrosine kinase inhibitors, epidermal growth factor tyrosine kinase inhibitors, mTOR inhibitors, and endothelial growth factor receptor 2 inhibitors (α-VEGFR-2 antibodies); various tyrosine kinase inhibitors such as MAP kinase inhibitors; cytokine-targeted inhibitors; proteasome inhibitors; and antibody-anticancer agent complexes. These inhibitors also include corresponding antibodies.

The mode of using the pharmaceutical agent of the present invention is not particularly limited. An appropriate mode of use can be selected according to the type of pharmaceutical agent. The pharmaceutical agent of the present invention can be used, for example, *in vitro* (for example, added to a culture medium of cultured cells) or *in vivo* (for example, administered to an animal), according to the purpose of use.

The target for application of the pharmaceutical agent of the present invention is not particularly limited. Examples of target mammals include humans, monkeys, mice, rats, dogs, cats, rabbits, pigs, horses, cows, sheep, goats, and deer. Examples of cells include animal cells and the like. The kind of cell is also not particularly limited. Examples of cells include blood cells, hematopoietic stem cells/progenitor cells, gametes (spermatozoa, oocytes), fibroblasts, epithelial cells, vascular endothelial cells, nerve cells, hepatocytes, keratinocytes, muscle cells, epidermal cells, endocrine cells, ES cells, iPS cells, tissue stem cells, cancer cells, and the like.

When the pharmaceutical agent of the present invention is used as an anticancer agent or applied to cancer cells, the target cancer is not particularly limited. Examples include hepatocellular carcinoma, pancreatic cancer, kidney cancer, leukemia, esophageal cancer, gastric cancer, colorectal cancer, lung cancer, prostate cancer, skin cancer, breast cancer, cervical cancer, and the like.

The pharmaceutical agent of the present invention can be in any dosage form. Examples of dosage forms include oral dosage forms, such as tablets (e.g., orally disintegrating tablets, chewable tablets, effervescent tablets, lozenges, and jelly-like drops), pills, granules, fine granules, powders, hard capsules, soft capsules, dry syrups, liquids (including health drinks, suspensions, and syrups), and jelly formulations; and parenteral dosage forms, such as injectable formulations (e.g., drip infusions (e.g., formulations for intravenous drip infusion), intravenous injections, intramuscular injections, subcutaneous injections, and intradermal injections), topical agents (e.g., ointments, plasters, and lotions), suppositories, inhalants, ophthalmic formulations, ophthalmic ointments, nasal drops, ear drops, and liposome formulations.

The administration route of the pharmaceutical agent of the present invention is not particularly limited, as long as the desired effect can be obtained. Examples include oral administration; parenteral administration including enteral administration, such as tube-feeding and enema administration, intravenous administration, intraarterial administration, intramuscular administration, intracardiac administration, subcutaneous administration, intradermal administration, and intraperitoneal administration; and the like.

The content of the active ingredient in the pharmaceutical agent of the present invention varies depending on, for example, the mode of use, the target of application, the condition of the target, etc.; and is not limited. For example, the content of the active ingredient is 0.0001 to 100 wt.%, and preferably 0.001 to 50 wt.%.

The dosage of the pharmaceutical agent of the present invention in the case of administration to an animal is not particularly limited, as long as it is a pharmaceutically effective amount. In the case of oral administration, in general, the dosage, in terms of the weight of the active ingredient, is usually 0.1 to 1000 mg/kg of body weight per day, and preferably 0.5 to 500 mg/kg of body weight per day. In the case of parenteral administration, the dosage is 0.01 to 100 mg/kg of body weight per day, and preferably 0.05 to 50 mg/kg of body weight per day. The above dosage can also be increased or decreased as appropriate depending on the age, disease state, symptoms, etc.

In addition to the medicaments and reagents, the lipid particle of the present invention can be used, for example, as a component of compositions, such as food additives, food compositions (including health foods, health enhancers, and nutritional products (e.g., supplements)), and cosmetics (also referred to herein as "the composition of the present invention"). Specifically, the lipid particle of the present invention can be used as compositions for anticancer and immunostimulatory uses. More specifically, the lipid particle of the present invention can be used as an active ingredient of type I INF production promoters, INF-α production promoters, Th1-related gene expression promoters, and the like.

The form of the composition of the present invention is not particularly limited, and can take a form generally used in each use according to the purpose of use.

Examples of the form of the composition of the present invention for use in food additives, health enhancers, nutritional products (e.g., supplements), and the like include tablets (including orally disintegrating tablets, chewable tablets, effervescent tablets, lozenges, and jelly-like drops), pills, granules, fine granules, powders, hard capsules, soft capsules, dry syrups, liquids (including suspensions and syrups), jelly formulations, and the like.

Examples of the form of the composition of the present invention for use in food compositions include liquid, gel, or solid foods, such as juices, soft drinks, teas, soups, soy milk, and other beverages; salad oils, dressings, yogurt, jellies, puddings, sprinkles, milk powder for childcare, cake mixes, dairy products (e.g. powder, liquid, gel, and solid), bread, confectionery (e.g., cookies), and the like.

The composition of the present invention may further contain one or more other components as necessary. These other components are not particularly limited, as long as they can be incorporated into food additives, food compositions, health enhancers, nutritional products (e.g., supplements), cosmetics, and the like. Examples include bases, carriers, solvents, dispersants, emulsifiers, buffers, stabilizers, excipients, binders, disintegrants, lubricants, thickeners, colorants, fragrances, chelating agents, and the like.

Other parts relating to the composition of the present invention are the same as those for the pharmaceutical agent of the present invention.

### Examples

The present invention is described in detail below based on Examples; however, the present invention is not limited by the Examples. The particle size of the lipid particles produced in the following Test Examples was 30 to 100 nm, except for those referred to as micro-size particles.

### Test Example 1. In Vitro Evaluation of Nucleic Acid/Cationic Lipid Complex

The induction of IFN-α production in human PBMC (peripheral blood mononuclear cells) by lipid nanoparticles containing an A-type CpG oligodeoxynucleotide (D35: SEQ No. 1: G_G_TGCATCGATGCAGGGG_G_G; the underline (_) indicates that nucleosides on both sides of the underline are phosphorothioate-bonded to each other; nucleosides that are not connected with an underline are phosphodiester-bonded to each other) containing a cationic lipid (#41: DOTAP, #45: DOTMA, or #47: DODAP) was examined. The specific procedures were as follows.

The content of each lipid per 100 mass% of lipids constituting the lipid particles is as follows: cationic lipid (#41: DOTAP, #45: DOTMA, or #47: DODAP): 50 mass%, DPPC: 19.5 mass%, cholesterol: 30 mass%, and DSPE-PEG (2k (PEG chain length))-OMe: 0.5 mass%. The ratio (N/P) of the number of nitrogen atoms (N) in the lipids constituting the lipid particles to the number of phosphorus atoms (D35) (P) in the nucleic acid was set to 3. A D35-containing aqueous solution and a lipid-containing ethanol solution were mixed using the NanoAssemblr (produced by Precision NanoSystems) to obtain lipid nanoparticles, which were then dialyzed against a 5% glucose aqueous solution. After the lipid nanoparticles (50 ng, 100 ng, or 200 ng, in terms of D35) were added to human PBMC culture medium, followed by culturing for 24 hours, the amount of IFN-α in the culture supernatant was quantified by the ELISA method.

The results are shown in Fig. 1. The ability to promote IFN-α production was highest when DOTAP was used as a cationic lipid (#41), followed by when DOTMA was used (#45) and when DODAP was used (#47). When DODAP was used, the stability was relatively low, and cloudy aggregation occurred within 3 months of storage at 4°C.

### Test Example 2. Examination of N/P Ratio

The induction of IFN-α production in human PBMC by D35-containing lipid nanoparticles with different N/P ratios was examined. The specific procedures were as follows.

The content of each lipid per 100 mass% of lipids constituting the lipid particles is as follows: cationic lipid (DOTAP): 50 mass%, DPPC: 19.5 mass%, cholesterol: 30 mass%, and DSPE-PEG (2k (PEG chain length))-OMe: 0.5 mass%. The ratio (N/P) of the number of nitrogen atoms (N) in the lipids constituting the lipid particles to the number of phosphorus atoms (D35) (P) in the nucleic acid was set to 3 (#41) or 1 (#42). A D35-containing aqueous solution and a lipid-containing ethanol solution were mixed using the NanoAssemblr (produced by Precision NanoSystems) to obtain lipid nanoparticles, which were then dialyzed against a 5% glucose aqueous solution. After the lipid nanoparticles (50 ng, 100 ng, or 200 ng, in terms of D35) were added to human PBMC culture medium, followed by culturing for 24 hours, the amount of IFN-α in the culture supernatant was quantified by the ELISA method.

The results are shown in Fig. 2. The ability to promote IFN-α production was higher when the N/P ratio was 3 (#41) than when the N/P ratio was 1 (#42). Further, when the N/P ratio was 1, the particle size was relatively large and was micro-scale.

### Test Example 3. Examination of Presence of pH-Sensitive Lipid and Effect of Dialysis Solution

The induction of IFN-α production in human PBMC by D35-containing lipid nanoparticles obtained by varying the dialysis solution with (or without) the addition of a pH-sensitive lipid (DOPE) was examined. The specific procedures were as follows.

The content of each lipid per 100 mass% of lipids constituting the lipid particles is as follows: cationic lipid (DOTAP): 50 mass%, DOPE or DPPC: 19.5 mass%, cholesterol: 30 mass%, and DSPE-PEG (2k (PEG chain length))-OMe: 0.5 mass%. The ratio (N/P) of the number of nitrogen atoms (N) in the lipids constituting the lipid particles to the number of phosphorus atoms (D35) (P) in the nucleic acid was set to 3. The combination of the presence of DOPE and the dialysis solution is as follows: #35: DOPE-free/PBS dialysis, #36: DOPE-free/5% glucose dialysis, and #38: DOPE-containing/5% glucose dialysis. A D35-containing aqueous solution and a lipid-containing ethanol solution were mixed using the NanoAssemblr (produced by Precision NanoSystems) to obtain lipid nanoparticles, which were then dialyzed against a 5% glucose aqueous solution or PBS. After the lipid nanoparticles (50 ng, 100 ng, or 200 ng, in terms of D35) were added to human PBMC culture medium, followed by culturing for 24 hours, the amount of IFN-α in the culture supernatant was quantified by the ELISA method.

The results are shown in Fig. 3. Fig. 3 revealed that the 5% glucose aqueous solution was desirable as the dialysis solution. Further, the ability to promote IFN-α production was higher when the pH-sensitive lipid was not contained.

### Test Example 4. Examination of Content and PEG Chain Length of DSPE-PEG-OMe

The induction of IFN-α production in human PBMC by D35-containing lipid nanoparticles obtained by varying the content and PEG chain length of DSPE-PEG-OMe (N-(methylpolyoxyethylene oxycarbonyl)-1,2-distearoyl-sn-glycero-3-phosphoethanolamine) was examined. The specific procedures were as follows.

The content of each lipid per 100 mass% of lipids constituting the lipid particles is as follows: cationic lipid (DOTAP): 50 mass%, DPPC (the content was adjusted so that the sum of DPPC and DSPE-PEG-OMe was 20 mass%), cholesterol: 30 mass%, and DSPE-PEG (2k or 5k (PEG chain length))-OMe: 0.0 mass%, 0.5 mass%, 1.0 mass%, 3.0 mass%, or 6.0 mass%. The ratio (N/P) of the number of nitrogen atoms (N) in the lipids constituting the lipid particles to the number of phosphorus atoms (D35) (P) in the nucleic acid was set to 3. The combination of the content and PEG chain length of DSPE-PEG-OMe is as follows: 2k PEG: 0.0 mass% (048), 2k PEG: 0.5 mass% (007), 2k PEG: 1.0 mass% (008), 2k PEG: 3.0 mass% (009), 2k PEG: 6.0 mass% (010), and 5k PEG: 0.5 mass% (011). A D35-containing aqueous solution and a lipid-containing ethanol solution were mixed using the NanoAssemblr (produced by Precision NanoSystems) to obtain lipid nanoparticles, which were then dialyzed against a 5% glucose aqueous solution or PBS. The lipid nanoparticles were added to human PBMC culture medium or BALB/c mouse spleen cell culture medium so that the medium final concentration in terms of D35 was 1 µg/mL, 0.5 µg/mL, or 0.25 µg/mL, followed by culturing for 24 hours. For the human PBMC culture medium, the amount of IFN-α in the culture supernatant was quantified by the ELISA method. For the mouse spleen cell culture medium, the amount of IFN-I in the culture supernatant was quantified by a biological activity measurement method using murine type I IFNs sensor cells (B16-Blue IFN-α/β cells).

The results are shown in Fig. 4. When 2k PEG was contained, the stability was highest and the biological activity was high at 0.5%, and the biological activity decreased as the PEG content increased. The biological activity decreased significantly at a PEG content of 3% or more. A comparison between 2k PEG 0.5% (007) and 5k PEG 0.5% (011) revealed that 2k PEG 0.5% showed higher biological activity.

### Test Example 5. Analysis of TLR9 Dependency

The TLR9 dependency of D35-containing lipid nanoparticles was examined. The specific procedures were as follows.

The content of each lipid per 100 mass% of lipids constituting the lipid particles is as follows: DOTAP: 50 mass%, DPPC: 19.5 mass%, cholesterol: 30 mass%, and DSPE-PEG (2k (PEG chain length))-OMe: 0.5 mass% or 3.0 mass%. The ratio (N/P) of the number of nitrogen atoms (N) in the lipids constituting the lipid particles to the number of phosphorus atoms (D35) (P) in the nucleic acid was set to 3. A D35-containing aqueous solution and a lipid-containing ethanol solution were mixed using the NanoAssemblr (produced by Precision NanoSystems) to obtain lipid nanoparticles, which were then dialyzed against a 5% glucose aqueous solution. In this Test Example and the following Test Examples, the D35-containing lipid nanoparticles obtained here were used.

Bone marrow cells were prepared from each of WT, TLR7KO, and TLR9KO mice, and stimulated with R848 (TLR7/8 agonist; medium final concentration: 1 ug/mL), D35 (TLR9 agonist; medium final concentration: 10 uM = 63.4 ug/mL), and D35-containing lipid nanoparticles (medium final concentration in terms of D35: 1 µg/mL), followed by culturing for 24 hours. Then, the amounts of IFN-I and IL-6 in the culture supernatant were quantified by a biological activity measurement method using murine type I IFNs sensor cells (B16-Blue IFN-α/β cells) and by the usual ELISA method, respectively.

The results are shown in Fig. 5. The IL-6 production stimulated with R848 disappeared in TLR7KO and did not disappear in TLR9KO, whereas the IL-6 production stimulated with D35 (TLR9 agonist) and with the D35-containing lipid nanoparticles (DSPE-PEG-OMe content: 0.5 mass% and 3.0 mass%) did not disappear in TLR7KO, but disappeared almost completely in TLR9KO. For IFN-I, similar results were shown, although the IFN-I production stimulated with R848 was very weak. Further, stimulation with D35 (10 uM = 63.4 ug/mL), which was basically not complexed with lipids, and stimulation with the D35-containing lipid nanoparticles (1 ug/mL) showed almost the same levels of IFN-I production; however, the IL-6 production stimulated with the D35-containing lipid nanoparticles (1 ug/mL) was about one-fourth that in the case of D35 (10 uM = 63.4 ug/mL). This indicated that the lipid nanoparticles promoted IFN-I production more than IL-6 production.

### Test Example 6. Examination with MC38 Mouse Colorectal Cancer-Derived Tumor Model-1

On days 9, 11, 13, 15, and 17 after subcutaneous transplantation of MC38 (1 x 10⁶ cells), a pharmaceutical agent (D35 or D35-containing lipid nanoparticles (DSPE-PEG-OMe content: 0.5 mass%)) was intratumorally administered 5 times in total. Then, the tumor size was measured. The amount of D35 nucleic acid administered at one time was adjusted to 4.5 ug/shot.

The results are shown in Fig. 6. With the D35-containing lipid nanoparticles, a significant decrease in tumor size was observed on days 17 and 21. No effect was observed with the administration of D35, which was not complexed with lipids.

### Test Example 7. Examination with MC38 Mouse Colorectal Cancer-Derived Tumor Model-2 (Intratumor Administration + Depletion)

On days 9, 11, 13, 15, and 17 after subcutaneous transplantation of MC38 (1 x 10⁶ cells), D35-containing lipid nanoparticles (DSPE-PEG-OMe content: 0.5 mass%) were intratumorally administered 5 times in total. Then, the tumor size was measured. Further, in order to deplete CD8T cells, anti-CD8a antibody (100 ug/shot) or control antibody (100 ug/shot) was intraperitoneally administered on days 6 and 13 after subcutaneous transplantation. The amount of D35 nucleic acid administered at one time was adjusted to 4.5 ug/shot.

The results are shown in Fig. 7. With the D35-containing lipid nanoparticles, a significant decrease in tumor size was observed on day 21. This decrease disappeared by depleting CD8T cells. This suggested that the anticancer effect of the D35-containing lipid nanoparticles depended on CD8T cells.

### Test Example 8. Examination with MC38 Mouse Colorectal Cancer-Derived Tumor Model-2 (Immune-Related Gene Evaluation)

mRNA was prepared from a frozen section of the tumor (on day 12 after subcutaneous transplantation) of Test Example 7, and the expression of various immune-related genes was measured by the qPCR method. As a result, the intratumoral administration of the D35-containing lipid nanoparticles increased the expression of many immune-related genes, and this increase disappeared by depleting CD8T cells. Further, since remarkable gene expression induction was observed in Th1-related genes (STAT4, T-bet, IFNg), it was suggested that the intratumoral administration of the D35-containing lipid nanoparticles resulted in tumor regression by shifting the intratumoral immune environment to Th1-type.

### Test Example 9. Examination with MC38 Mouse Colorectal Cancer-Derived Tumor Model-3 (Intravenous Administration)

On days 9, 11, 13, 15, and 17 after subcutaneous transplantation of MC38 (1 x 10⁶ cells), a pharmaceutical agent (D35 or D35-containing lipid nanoparticles (DSPE-PEG-OMe content: 0.5 mass%)) was administered intravenously 5 times in total. Then, the tumor size was measured. The amount of D35 nucleic acid administered at one time was adjusted to 25 ug/shot.

The results are shown in Fig. 8. With the D35-containing lipid nanoparticles, a significant decrease in tumor size was observed. This revealed that the D35-containing lipid nanoparticles could exert an anticancer effect even when administered intravenously.

### Test Example 10. Examination with MC38 Mouse Colorectal Cancer-Derived Tumor Model-3 (Immune-Related Gene Evaluation)

mRNA was prepared from a frozen section of the tumor (on day 12 after subcutaneous transplantation) of Test Example 9, and the expression of various immune-related genes was measured by the qPCR method. As a result, increased expression of Ifng and CXCL9, which suggested intratumoral immune activation, was observed. This suggests that intravenous administration causes the same immune changes in the tumor as intratumoral administration.

### Test Example 11. Examination with MC38 Mouse Colorectal Cancer-Derived Tumor Model-4 (Intratumoral Administration + Depletion)

On days 9, 11, 13, 15, and 17 after subcutaneous transplantation of MC38 (1 x 10⁶ cells), D35-containing lipid nanoparticles (DSPE-PEG-OMe content: 0.5 mass%) were administered intravenously 5 times in total. Then, the tumor size was measured. Further, in order to deplete CD8T cells, anti-CD8a antibody (100 ug/shot) or control antibody (100 ug/shot) was intraperitoneally administered on days 6 and 13 after subcutaneous transplantation. The amount of D35 nucleic acid administered at one time was adjusted to 25 ug/shot.

The results are shown in Fig. 9. With the D35-containing lipid nanoparticles, a significant decrease in tumor size was observed on day 21. This decrease disappeared by depleting CD8T cells. This suggested that the anticancer effect of the D35-containing lipid nanoparticles depended on CD8T cells.

### Test Example 12. Examination with MC38 Mouse Colorectal Cancer-Derived Tumor Model-5 (Intratumoral Administration + Anti-PD-1_ Antibody)

On days 9, 11, 13, 15, and 17 after subcutaneous transplantation of MC38 (1 x 10⁶ cells), a pharmaceutical agent (D35-containing lipid nanoparticles (DSPE-PEG-OMe content: 0.5 mass%), anti-PD-1 antibody, or a combination thereof) was administered 5 times in total. Then, the tumor size was measured. The D35-containing lipid nanoparticles were administered intratumorally, and the anti-PD-1 antibody was administered intraperitoneally. The amount of D35 nucleic acid administered at one time was adjusted to 4.5 ug/shot.

The results are shown in Fig. 10. The combination of D35-containing lipid nanoparticles and anti-PD-1 antibody showed a stronger anticancer effect than monotherapy using each of them alone.

### Test Example 13. Examination with MC38 Mouse Colorectal Cancer-Derived Tumor Model-5 (Intravenous Administration + Anti-PD-1 Antibody)

On days 9, 11, 13, 15, and 17 after subcutaneous transplantation of MC38 (1 x 10⁶ cells), a pharmaceutical agent (D35-containing lipid nanoparticles (DSPE-PEG-OMe content: 0.5 mass%), anti-PD-1 antibody, or a combination thereof) was administered 5 times in total. Then, the tumor size was measured. The D35-containing lipid nanoparticles were administered intravenously, and the anti-PD-1 antibody was administered intraperitoneally. The amount of D35 nucleic acid administered at one time was adjusted to 25 ug/shot.

The results are shown in Fig. 11. The combination of D35-containing lipid nanoparticles and anti-PD-1 antibody showed a stronger anticancer effect than monotherapy using each of them alone.

### Test Example 14. Toxicity Evaluation

Mice (C57BL/6J) were intravenously administered with D35 or D35-containing lipid nanoparticles (DSPE-PEG-OMe content: 0.5 mass% or 3.0 mass%). One day after administration, blood was collected and paraffin sections of the liver were prepared. The ALT and AST levels in blood were measured with a measurement kit (Transaminase CII-test Wako, produced by FUJIFILM Wako Pure Chemical Corporation). Further, the paraffin sections were stained with HE to determine the occurrence of erythrocyte hemolysis.

As a result, the ALT and AST levels in blood with administration of the D35-containing lipid nanoparticles were equivalent to those without administration thereof. In addition, no erythrocyte hemolysis was observed.

## Claims

1. A lipid particle comprising an A-type CpG oligodeoxynucleotide.

2. The lipid particle according to claim 1, wherein the lipid particle comprises an outer layer and an ion complex placed inside the outer layer.

3. The lipid particle according to claim 2, wherein the ion complex contains an A-type CpG oligodeoxynucleotide.

4. The lipid particle according to claim 2 or 3, wherein the outer layer is a lipid monolayer membrane in which amphipathic lipids are arranged with hydrophilic parts facing outward.

5. The lipid particle according to any one of claims 1 to 4, wherein a water-soluble polymer-modified lipid is contained in an amount of 0 to 10 mass% based on 100 mass% of lipids constituting the lipid particle.

6. The lipid particle according to any one of claims 1 to 5, wherein the ratio (N/P) of the number of nitrogen atoms (N) in the lipids constituting the lipid particle to the number of phosphorus atoms (P) in a nucleic acid containing the A-type CpG oligodeoxynucleotide is 2.5 or more.

7. The lipid particle according to any one of claims 1 to 6, comprising a cationic lipid.

8. The lipid particle according to claim 7, wherein the cationic lipid is at least one member selected from the group consisting of 1,2-dioleoyloxy-3-trimethylammonium-propane (DOTAP) and 1,2-dioleyloxy-3-trimethylammonium-propane (DOTMA).

9. A method for producing a lipid particle, comprising the step of mixing a lipid-containing alcohol solution and an A-type CpG oligodeoxynucleotide-containing aqueous solution.

10. The production method according to claim 9, wherein the step is performed in a reaction system using a microchannel.

11. A medicament comprising the lipid particle according to any one of claims 1 to 8.

12. A reagent comprising the lipid particle according to any one of claims 1 to 8.

13. An immunostimulant comprising the lipid particle according to any one of claims 1 to 8.

14. An anticancer agent comprising the lipid particle according to any one of claims 1 to 8.
